Europäisches Patentamt

(19) European Patent Office

Office européen des brevets

(11) Publication number: 0 190 057
A1

# (12) EUROPEAN PATENT APPLICATION

(21) Application number: 86300683.9

(22) Date of filing: 31.01.86

(51) Int. Cl.⁴: C 07 C 2/68
C 07 C 15/073

(30) Priority: 31.01.85 GB 8502390

(43) Date of publication of application:
06.08.86 Bulletin 86/32

(84) Designated Contracting States:
BE DE FR GB IT NL

(71) Applicant: THE DOW CHEMICAL COMPANY
2030 Dow Center Abbott Road P.O. Box 1967
Midland, MI 48640(US)

(72) Inventor: Steenwinkel, Michael De
Spuikompark 1
4553 BG Philippine(NL)

(72) Inventor: Schoof, Johannes Wilhelmus Maria
Trompstraat 1
4535 BT Terneuzen(NL)

(74) Representative: Raynor, John et al,
W.H. Beck, Greener & Co 7 Stone Buildings Lincoln's Inn
London WC2A 3SZ(GB)

(54) Process for the alkylation of aromatic hydrocarbons.

(57) In the production of alkylated aromatics, particularly ethylbenzene, using Friedel Crafts catalysts, the yield is improved by treating the residue with fresh Friedel Crafts catalyst, which is then introduced into the alkylation line and is still sufficiently active to take part in the main alkylation process. Preferably, on a light fraction of the residue is utilized.

0190057

PROCESS FOR THE ALKYLATION OF AROMATIC HYDROCARBONS

This invention relates to the alkylation of aromatic hydrocarbons, particularly benzene, with an alkylating agent, for example ethylene or propylene, and a catalyst of the so called "Friedel Crafts" type. Alkylation processes are used extensively in industry, for example for the production of ethylbenzene and cumene from benzene, by reaction with ethylene or propylene, respectively.

Ethylbenzene, which is produced by such an alkylation reaction, is a raw material for a great many organic chemicals and many millions of tons are produced annually throughout the world.

The alkylation reaction leads to an equilibrium and the desired mono-alkylated aromatic compound is only one of the many products present in the reaction mixture at equilibrium.

In the reaction of benzene with ethylene in the presence of aluminium trichloride to produce ethylbenzene,

31,209-F                    -1-

0190057

the reaction mixture leaving the reactor will commonly have a composition about as follows:

| | |
|---|---|
| Benzene | about 50 percent |
| Ethylbenzene | about 37 percent |
| Diethylbenzene | about 10 percent |
| Triethylbenzene | about 1.5 percent |

The remainder is constituted by heavier components, for example polyphenylated ethanes, polyalkylated benzenes and other higher hydrocarbons.

In order to obtain the desired ethylbenzene in a suitable yield, the reaction mixture from the reactor is passed to a series of distillation columns, to enable the various constituents to be recovered. Typically, a first distillation column will extract benzene from the mixture, a second will extract the desired ethylbenzene, and a third will extract di- and tri- ethylbenzenes.

Because the reactions of di- and tri- ethylbenzenes to mono- and di- ethylbenzenes (transalkylation) are reversible, di- and tri- ethylbenzene will react further in the presence of the Friedel Crafts catalyst and additional benzene, to produce further ethylbenzene until equilibrium is again reached. They are therefore returned to the reactor.

The bottom residue from the third distillation column is a mixture, comprising polyphenyl ethanes, polyalkylated benzene, as well as other substances.

Conventionally, the yield of the overall process is improved by recycling at least part of this residue to the reactor although it is found that this tends to lead to the increased build up of heavy components and tars in the alkylation system and significant deactivation of the catalyst utilized.

U.K. Patent Specification No. 794570 discloses a method for the alkylation of benzene with propylene to produce cumene, in which the bottom residue from the distillation of the reaction products is treated with fresh Friedel Crafts catalyst to promote transalkylation of the residue and produce more of the desired product. In the method disclosed in this British patent, however, once the catalyst has been utilized in the alkylation process, it is not suitable for further use, and rather than being recycled, it is discarded.

We have now found that a substantial improvement in the overall yield in the alkylation of aromatic hydrocarbons, and particularly in the alkylation of benzene with ethylene, can be obtained, together with substantial improvements in catalyst economy, if the catalyst is separated from the mixture of reaction products in a settling vessel, and at least a proportion, and preferably substantially all of the separated catalyst is returned to the reactor to take further part in the alkylation reaction, and if the alkylation residue, or preferably a lighter fraction thereof, is treated with fresh Friedel Crafts catalyst which is then further utilized in the alkylation reaction. Fresh catalyst is a complex generally prepared by the reaction of aluminium trichloride, one or more aromatic hydrocarbons, (for example, diethylbenzene or ethylbenzene) and any other co-promoter, for example HCl and/or ethyl chloride.

0190057

Surprisingly, the use of such fresh catalyst in this way to cause the trans-alkylation (that is to say the migration of alkyl groups from one molecule to another) of the relatively heavy residues, followed by the use of the catalyst in the main alkylation process and subsequent recycling of the catalyst to the reactor, results in a substantial improvement in yield and also improvement in catalyst economy.

The alkylation residue reacts with the fresh catalyst in a two phase mixture, and this reaction mixture is allowed to settle. The upper layer of the settled mixture comprises essentially alkylated aromatics, and the lower layer is a catalyst layer. The catalyst layer has an activity which is still sufficient to enable it to be used as a partial or total replacement, preferably a total replacement, of fresh Friedel Crafts catalyst, for the alkylation process.

The catalyst which has been utilized for trans-alkylation of the residue can therefore be introduced into the alkylation line, upstream of the distillation vessels and preferably into the reaction vessel.

Accordingly, in a first aspect of the present invention, there is provided a process for the alkylation of an aromatic compound, which process comprises:
reacting the aromatic compound with an alkylating agent in the presence of a Friedel Crafts catalyst in a reactor,
passing the reaction mixture from the reactor to a settling vessel to allow separation of the Friedel Crafts catalyst from the mixture of reaction products,

- 5 -

recycling at least a proportion of the separated Friedel Crafts catalyst to the reactor to take further part in the alkylation reaction,

separating the desired alkylated aromatic compound from the mixture of reaction products in a distillation column,

reacting the residue of the alkylation process with a portion of fresh Friedel Crafts catalyst to cause trans-alkylation thereof, and

subsequently introducing the catalyst after its utilization in the trans-alkylation reaction into the alkylation line upstream of the distillation column.

The said catalyst which is not a recycled catalyst, but is fresh apart from having been used in the trans-alkylation reaction is preferably introduced into the alkylation line in the reactor itself, where it is effective to catalyze further alkylation of the preferred benzene starting material. It can there be mixed with the recycled catalyst from the settling vessel in order to raise the activity of the total catalyst stream.

The mixture of reaction products from the settling vessel is preferably passed through at least three distillation columns, a first distillation column for separating unreacted aromatic compounds, e.g., benzene, a second distillation column for separating mono-alkylated product, e.g., mono-alkylated benzene, and a third distillation column, for separating primarily di- and tri- alkylated products, e.g., di- and tri- alkylated benzene.

The bottom residue from the third distillation column, or preferably, a light fraction thereof, is then treated with the fresh Friedel Crafts catalyst, as indicated above. The resulting catalyst, which is still active, is then introduced into the alkylation line, upstream of the distillation column, and preferably into the reactor, although it is also possible to introduce it into the settling vessel, where some further equilibration generally takes place. The treated residue will generally also be returned to the alkylation line upstream of the distillation columns, preferably into the reactor, with or without prior separation from the catalyst. It is generally preferred to separate the treated residue from the catalyst, and then introduce both into the reactor in separate streams, since this facilitates accurate process control.

The process is particularly useful for the alkylation of mononuclear aromatics, such as toluene and xylenes, and particularly, benzene although it can also be used for polynuclear aromatics, such as naphthalene.

A particular embodiment of the invention will now be described with reference to the accompanying drawings, in which:-

Figure 1 is a schematic diagram of a conventional alkylation line for the production of ethylbenzene incorporating a modification in accordance with the invention, and,

Figure 2 illustrates additional components for carrying out a preferred embodiment of the method accordingly to the invention.

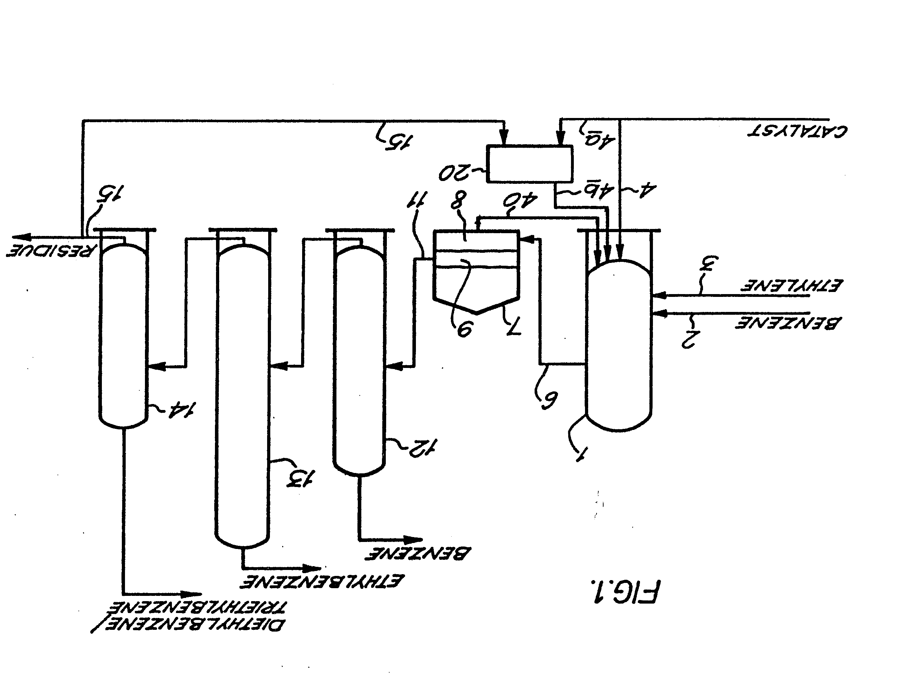

Referring first to Figure 1, a conventional plant for the alkylation of benzene comprises a reactor 1, fed by lines 2, 3 and 4, for supplying benzene, ethylene, and the Friedel Crafts catalyst. Methods for preparing such catalysts from $AlCl_3$ and aromatic compounds such as diethylbenzene and ethylbenzene are very well known from the prior art.

The alkylation reaction is exothermic, and is generally maintained at a temperature of approximately 100° to 200°C.

The mole ratio of ethylene to benzene will generally be from 0.3 to 0.9, preferably from 0.4 to 0.6.

The catalyst is generally used in an amount of from 0.0001 to 0.01 moles (calculated as $AlCl_3$), preferably from 0.0005 to 0.007 moles, more preferably from 0.001 to 0.003 moles, per mole of ethylene.

The reaction mixture leaves the reaction vessel through a line 6, and is introduced into a settling tank 7, in which the reaction mixture separates into a lower phase 8, containing Friedel Crafts catalyst, which has been at least partially deactivated and an upper phase 9, containing substantially benzene and alkylated benzenes.

The lower catalyst phase 8 is still active, and may be reintroduced into the reaction vessel 1 in line 40, to promote further alkylation of benzene. It is not however sufficiently active for reaction with the heavy residue of the alkylation reaction, as will be described hereinafter. Fresh catalyst must be used for this purpose in accordance with the method of the invention.

The upper phase 9 is passed through a further line 11 to a distillation section.

The distillation section consists of three distillation columns, 12, 13 and 14, of conventional form. In the first distillation column 12, benzene is separated and recycled to the reaction vessel 1.

31,209-F        -7-

The bottom fraction from the distillation column 12 is passed to a second distillation column 13, for the separation of the desired ethylbenzene.

The bottom fraction from column 13 is passed to a third distillation column 14, for the separation primarily of di- and tri- ethylbenzenes.

The bottom fraction (residue) of column 14 exits via a line 15 and is passed in accordance with the invention to a mixing chamber 20, where it is allowed to react using a quantity of fresh catalyst which enters chamber 20 by means of line 4a. The catalyst causes a substantial degree of trans-alkylation of the residue and the mixture of products is passed to reactor 1 via line 4b.

A number of embodiments of the invention will now be described in the following examples:

Example 1

The residue from line 15 was treated in a vacuum stripper at 230° to 265°C on a laboratory scale to produce a light fraction and a heavy tar fraction.

Fresh catalyst was prepared by the reaction of aluminium trichloride ($AlCl_3$), and excess of an aromatic hydrocarbon, and a co-promoter. Two test samples were prepared, containing amounts of the mixture of fresh catalyst (both complex and hydrocarbon phase) and the heavy and light fractions as shown in Table I.

## TABLE I

| | Cata | lyst | | |
| | Complex Phase | Hydrocarbon Phase | Heavy Residue | Light Residue |
|---|---|---|---|---|
| Test 1 | 102 g | 440 g | 39.4 | 37.6 |
| Test 2 | 106 g | 445 g | -- | 37.1 |

Each of the test samples were analyzed for benzene, ethylbenzene, and other constituents as shown in Table II, both initially, and after having been allowed to react for a period of 120 minutes at 60°C. Table 2 shows the composition of the two test samples both before and after the 120 minute period. The activity of the catalyst in the test samples after the 120 minute period was found to be still sufficient to enable the catalyst to catalyze the benzene alkylation reaction.

## Table II

| W/W% | Test Sample 1 | | Test Sample 2 | |
| | Initial | Final | Initial | Final |
|---|---|---|---|---|
| Benzene | 61.6 | 55.6 | 69.3 | 68.3 |
| Ethylbenzene | 8.0 | 16.9 | 9.1 | 17.4 |
| Diethylbenzene | 2.0 | 1.4 | 2.2 | 1.2 |
| Triethylbenzene | 2.1 | 0.6 | 2.3 | 0.6 |
| Residue | 23.3 | 23.1 | 14.4 | 11.0 |

It can be seen that the treatment with fresh catalyst results in significant conversion to ethylbenzene. Furthermore, although the activity of the catalyst is lowered, it is still sufficiently high to enable it to take part in the alkylation reaction.

31,209-F                    -9-

In one aspect of the invention, the residue from vessel 14 may be treated directly with fresh Friedel Crafts catalyst, and returned to the reaction vessel 1. In a preferred embodiment, however, the residue from line 15 may first be separated into light and heavy fractions, and only one of these, preferably the light fraction, treated in accordance with the invention.

Example 2

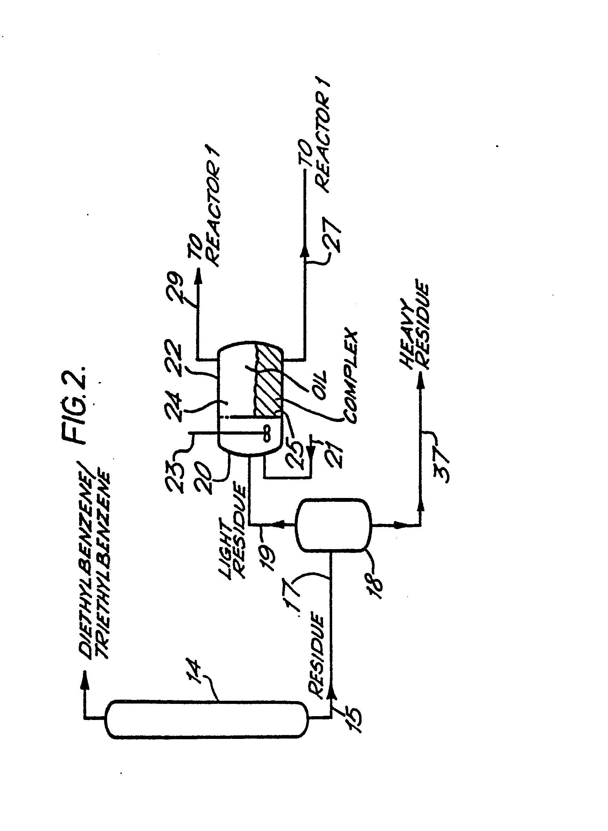

This example illustrates the practical application of a preferred embodiment to a line for the production of ethylbenzene, as illustrated in Figure 2.

In the embodiment shown in Figure 2, the residues from line 15 of Figure 1 are passed via a line 17 to a residue flash drum 18. The residue flash drum 18 separates the residue mixture into a lighter fraction, containing primarily higher alkylated benzenes and similar products, and a heavier, tarry fraction.

The lighter fraction from the residue flash drum 18 is passed via a line 19 to a mixing chamber 20, where it is mixed with fresh Friedel Crafts catalyst, which is introduced via line 21. A mixing paddle 23 is provided for this purpose. The fresh catalyst entering via line 21 is produced in a separate catalyst make-up system (not shown) of conventional type.

The residence time in the mixing chamber is generally from 1 minute to 3 hours at a temperature of from 40° to 200°C with higher temperatures requiring lower residence time.

The mixture overflows from the mixing chamber 20 into a settling chamber 22, in which separation takes

31,209-F                    -10-

place into an upper phase 24, containing primarily benzene alkylation products, and a lower phase 25, comprising the catalyst. The phase separation makes it possible to feed the lower phase 25 containing the catalyst to the alkylation line in a controlled way.

For a typical ethylbenzene line, the relative flow rates of the light tars fraction in line 19, and the fresh catalyst in line 21 would be of the order of from 0 to 1, preferably from 0.4 to 0.8, most preferably 0.5 to 0.7.

The upper phase 24 containing trans-alkylated benzenes, including a substantial quantity of the desired ethylbenzene, may be reintroduced into the alkylation line via line 29 at any convenient point, for example after the separation vessel 7 and preferably into the reactor 1. The lower phase 25 is introduced via line 27 into the reactor 1.

The heavy residue from the residue flash drum 18 is passed to waste via line 37.

It is to be noted that Friedel Crafts catalyst which has been used once, for example that forming the lower phase in the vessel 7, is not sufficiently active to cause substantial trans-alkylation of the heavy residue. Fresh Friedel Crafts catalyst however is capable of completing the trans-alkylation reaction within about a half hour.

It was found that line 19 contained no benzene or ethylbenzene. The catalyst (both complex and oil) was fed via line 21 and contained 19.5 percent ethylbenzene. The oil phase exiting vessel 22 via line 29 contained 24 percent ethylbenzene, while the calculated ethylbenzene concentration of the feedstreams was 16 percent. The catalyst exiting from chamber 22 via line 27 had an activity which was less than that of the fresh catalyst introduced into chamber 20, but was still sufficiently active to cause further alkylation when returned to the reactor 1.

Example 3

In a second run using the same set-up as in Example 2, line 19, contained no benzene or ethylbenzenes and 2 percent diethylbenzene. Line 21 (catalyst) contained 35 percent ethylbenzene. After reaction, line 29 contained 38 percent ethylbenzene and 9 percent diethylbenzene, the remainder being other aromatic hydrocarbons.

The activity of the catalyst exiting from chamber 22 was still sufficiently high to be useful in the reaction in reactor 1.

It can be seen from Examples 2 and 3 that the method of the invention makes possible a small but significant improvement in yield in an otherwise conventional ethylbenzene production line.

Utilizing the method of the invention, the overall yield for the ethylation of benzene can be improved from about 98.5 percent to something approaching 99.1 percent or higher. Although this improvement in yield may seem small, it is very significant when viewed in the context of the scale of world ethylbenzene production.

It will of course be appreciated that various other particular arrangements are possible, other than

**0190057**

those shown in the accompanying drawings. In particular, the treated residue may be reintroduced into the line at any convenient point, and various catalyst to residue ratios may be utilized.

Claims:

1. A process for the alkylation of an aromatic compound, which process comprises:

reacting the aromatic compound with an alkylating agent in the presence of a Friedel Crafts catalyst in a reactor,

passing the reaction mixture from the reactor to a settling vessel to allow separation of the Friedel Crafts catalyst from the mixture of reaction products,

recycling at least a proportion of the separated Friedel Crafts catalyst to the reactor to take further part in the alkylation reaction,

separating the desired alkylated aromatic compound from the mixture of reaction products in a distillation column,

reacting the residue of the alkylation process with a portion of fresh Friedel Crafts catalyst to cause trans-alkylation thereof, and

subsequently introducing the catalyst after its utilization in the trans-alkylation reaction into the alkylation line upstream of the distillation column.

2. A process as claimed in Claim 1, wherein the aromatic compound is a mono-nuclear aromatic compound.

3. A process as claimed in Claim 2, wherein the aromatic compound is benzene, xylene or toluene.

4. A process as claimed in any one of the preceding claims, wherein the reaction mixture from the reactor is passed to at least three distillation columns, a first distillation column for separating unalkylated aromatic compounds, a second distillation column for separating mono-alkylated product, and a third distillation column for separating primarily di- and tri- alkylated products.

5. A process as claimed in Claim 4, wherein the residue from the third distillation column is treated directly with the fresh Friedel Crafts catalyst, and reintroduced into the process line upstream of the first distillation column.

6. A process as claimed in Claim 4, wherein the residue from the third distillation column is separated into a heavy fraction and a light fraction and only the light fraction is treated with fresh Friedel Crafts catalyst, and reintroduced into the process line upstream of the first distillation column.

7. A process as claimed in any one of the preceding claims, wherein the catalyst used to treat the residue is introduced after such treatment into the reactor.

8. A process as claimed in any one of the preceding claims, wherein the alkylating agent is ethylene.

9.    A process as claimed in Claim 1, 4 or 7, wherein the aromatic compound is benzene and the alkylating agent is ethylene.

FIG.1.

DIETHYLBENZENE/
TRIETHYLBENZENE

ETHYLBENZENE

BENZENE

1

6

2 BENZENE

ETHYLENE

3

7

9

12

13

14

RESIDUE

4 4b

40

8

11

15

CATALYST

4a

20

15

0190057

FIG.2.

DIETHYLBENZENE/
TRIETHYLBENZENE

LIGHT
RESIDUE

RESIDUE

HEAVY
RESIDUE

TO
REACTOR 1

TO
REACTOR 1

OIL

COMPLEX

## DOCUMENTS CONSIDERED TO BE RELEVANT

EP 86300683.9

| Category | Citation of document with indication, where appropriate, of relevant passages | Relevant to claim | CLASSIFICATION OF THE APPLICATION (Int. Cl.4) |
|---|---|---|---|
| A | DE - A1 - 3 009 632 (BASF)<br>* Page 2, line 31 - page 7, line 24 * | 1-4,6, 8,9 | C 07 C 2/68<br>C 07 C 15/073 |
| A | DE - A1 - 3 039 760 (BASF)<br>* Page 5, line 7 - page 8, line 19 * | 1-4,8, 9 | |
| D,A | GB - A - 794 570 (ICI)<br>* Claims 1,4,11,14 * | 1-3,5, 7 | |
| A | GB - A - 639 873 (MONSANTO)<br>* Page 2, line 113 - page 4, line 36 * | 1-3,7- 9 | |

| | |
|---|---|
| | TECHNICAL FIELDS SEARCHED (Int. Cl.4) |
| | C 07 C 2/00<br>C 07 C 15/00 |

The present search report has been drawn up for all claims

| Place of search | Date of completion of the search | Examiner |
|---|---|---|
| VIENNA | 17-04-1986 | KÖRBER |